Europäisches Patentamt

European Patent Office

Office européen des brevets

(11) Publication number: **0 133 052**
A2

# EUROPEAN PATENT APPLICATION

(21) Application number: 84305214.3

(22) Date of filing: 31.07.84

(51) Int. Cl.⁴: **B 01 J 37/30**
**B 01 J 23/74, C 07 C 2/10**
**C 07 C 2/12**

(30) Priority: 01.08.83 US 519297

(43) Date of publication of application:
13.02.85 Bulletin 85/7

(84) Designated Contracting States:
BE DE FR GB IT NL

(71) Applicant: CONOCO INC.
1000 South Pine Street P.O. Box 1267
Ponca City Oklahoma 74601(US)

(72) Inventor: Higley, David P.
1113 Princeton
Ponca City Oklahoma 74601(US)

(72) Inventor: Dowling-Davis, Robin M.
P.O. Box 20462
Bloomington Minnesota 55420(US)

(72) Inventor: Yang, Kang
2501 Robin Road
Ponca City Oklahoma 74601(US)

(72) Inventor: Connelly-Cremers, Martha
548 Virginia Avenue
Ponca City Oklahoma 74601(US)

(74) Representative: Woodman, Derek et al,
Frank B. Dehn & Co. European Patent Attorneys Imperial
House 15-19 Kingsway
London WC2B 6UZ(GB)

(54) An Ni+2 exchange silica-alumina catalyst for olefin oligomerization.

(57) A non-impregnated, chemically incorporated $Ni^{+2}$ silica-alumina catalyst for the oligomerization of olefins is made by a process comprising: (a) contacting silica-alumina $[SiO_2/Al_2O_3]$ with a molar excess of $Ni^{+2}$ cations for a sufficient length of time to permit an essentially complete chemical reaction between $Ni^{+2}$ cations and the accessible reactive sites in the silica-alumina, (b) washing the solid catalyst of step (a) in an effective amount of water for a sufficient length of time to remove substantially all unbound $Ni^{+2}$ cations present after step (a); and (c) drying and then calcining the solid catalyst remaining after step (b) by heating to a temperature of from about 300°C to about 700°C.

A Ni$^{+2}$ EXCHANGE SILICA-ALUMINA CATALYST 0133052
FOR OLEFIN OLIGOMERIZATION

## The Prior Art

Oligomerization and polymerization of olefins are frequently conducted over catalysts prepared by impregnation of a catalyst support using a nickel salt solution, followed by drying; decomposition of the nickel salt to nickel oxide, and activation of the catalyst. Processes and catalysts such as these are found in U.S. Patents 2,949,429; U.S. Patent 3,045,054; U.S. Patent 2,904,608; and U.S. Patent 3,341,620.

"Impregnate" is defined as the incorporation of one substance into another, usually in a finely divided, or a finely separated form (definition taken from the Van Nostrand Chemists' Dictionary, 1953, publisher D. Van Nostrand Co. Inc). A nickel catalyst prepared by impregnation thus is a catalyst wherein the nickel salt is physically incorporated into the support's pores, and on top of the support's surface.

A consequence of such impregnation methods can be a catalyst having much more nickel than is needed for peak activity of the catalyst. Moreover, this build up of nickel salt on the surface of the solid support can decrease catalyst surface area. It would be advantageous to prepare a solid, fixed-bed nickel catalyst for olefin oligomerization which consumes less nickel but delivers greater activity. It is thus an object of the instant invention to provide a fixed-bed nickel catalyst for olefin oligomerization which is superior in activity, and advantageously consumes minimum amounts of nickel. Other objects will become apparent to those skilled in the art as this dicussion proceeds.

## Summary of the Invention

A non-impregnated, chemically incorporated Ni$^{+2}$ silica-alumina catalyst for the oligomerization of

olefins is made by a process comprising: (a) contacting silica-alumina [$SiO_2$-$Al_2O_3$] with molar excess of $Ni^{+2}$ cations for a sufficient length of time to permit an essentially complete chemical reaction between $Ni^{+2}$ cations and the accessible reactive sites in the silica-alumina, (b) washing the solid catalyst of step (a) in an effective amount of water for a sufficient length of time to remove substantially all unbound $Ni^{+2}$ cations from step (a); and, (c) drying and then calcining the solid catalyst remaining after step (b) by heating it to a temperature of from about 300°C to about 700°C.

The most critical step in this process is washing the solid catalyst after it has been removed from the aqueous solution of the nickel salt. Thoroughly washing the solid catalyst after exposure to the aqueous solution assures that substantially all of the nickel present in the catalyst is chemically incorporated and is part of the actual structure of the solid catalyst. The presence of nickel in the form of a salt or elemental nickel merely resting on or supported by the solid silica-alumina is undesirable. Surprisingly, although less nickel is needed for the catalyst of the instant invention, a more active catalyst results than would be provided by impregnation.

The washing must be sufficient to remove substantially all of the unreacted nickel cations from the solid catalyst. Generally the amount of water used for this washing step, and the length of time that the solid catalyst is washed will depend upon the amount of solid catalyst, and the concentration of the solution of nickel ions that the silica-alumina contacted. After washing removes all of the unreacted nickel, the catalyst preparation is completed by drying and calcining the remaining solid.

After the preparation of the solid catalyst, it can be used to oligomerize lightly branched and/or linear olefins by a process comprising: contacting the catalyst with lightly branched and/or linear olefins having from 2 to about 15 carbon atoms per molecule, at temperatures in the range of about 10°C to about 200°C and under pressures in the range of from about one to about 75 atmospheres, and then collecting the oligomerized product.

## Detailed Description

The aqueous nickel salt solution should be sufficiently concentrated to insure that an effective amount of $Ni^{+2}$ cations contact the silica-alumina and provide rapid, substantially complete reaction. Furthermore, there should be a sufficient amount of solution to completely immerse the silica-alumina. Acceptably the catalyst can be made by immersing one volume of solid silica-alumina in one portion of a nickel salt solution where the volume of said solution is equal to at least twice the volume of silica-alumina used in a minimum concentration of about .05 molar. To insure rapid and complete reaction, a preferable minimum concentration is .3 molar. The more concentrated solution is preferred since a complete reaction between the nickel and the $[SiO_2-Al_2O_3]$ will be achieved faster and result in the chemical incorporation of more nickel into the silica-alumina matrix. A complete reaction will give the maximum number of olefin reactive sites possible without decreasing the effectiveness of the catalyst by the presence of deposited nickel salt, or metallic nickel on the surface of the catalyst. Representative but non-exhaustive examples of useful nickel salts are nickel sulfate, nickel chloride, nickel nitrate and mixtures of these. The silica-alumina is immersed in the aqueous nickel salt solution for a sufficient length of time to insure a complete reaction between the nickel ions and the $[SiO_2-Al_2O_3]$. This length of time will decrease as the concentration of solution increases.

Normally, the solid silica-alumina is exposed to the nickel solution for at least 30 seconds. The maximum length of time is not critical, since the factor to be considered is whether or not the reaction has gone to completion. The $Ni^{+2}$ cations can be contacted with the silica-alumina preferably in excess of five minutes. An alternative to allowing the solid $[SiO_2-Al_2O_3]$ to remain in contact with the nickel salt solution for an extended length of time is to repeat the exposure of the solid catalyst to another solution of nickel salt after the solid catalyst has been dried, or just after washing and before drying. Preferably, the sequence of exposing the solid silica-alumina to $Ni^{+2}$ ions and then washing the solid catalyst is repeated once after the first sequence; and most preferably the sequence is repeated at least twice to insure complete reaction.

A critical step in the catalyst preparation is the washing of the solid catalyst after exposure of the silica-alumina to the aqueous nickel salt solution. This washing must be done for a sufficient length of time in a sufficient amount of water to remove all the unreacted nickel cations from the presence of the $[SiO_2-Al_2O_3]$. This insures that the only nickel species remaining within the solid catalyst is chemically reacted with the $[SiO_2-Al_2O_3]$. Normally, the solid catalyst will be washed in a sufficient amount of water to completely immerse the solid catalyst for a minimum of 30 seconds. Acceptably the washing step can be accomplished by washing with 10 volumes of wash $H_2O$ per volume of catalyst. When using this 10 vol/1 vol ratio, preferably, agitation is used during washing. A more preferred and effective wash is accomplished through the ratio of 15 volumes of wash water per volume of catalyst with a minimum agitated wash time of 1 minute. The most preferred wash step uses at least 2 sequentially applied volumes of wash water. The wash water may optionally be basic with a pH of from about 7.2 to about 12. The preferred base is ammonium hydroxide. Most preferably, the solid catalyst is agitated when immersed in the wash water.

An optional procedure for the catalyst preparation, which encourages the reaction of the $Ni^{+2}$ ion and thus

- 5 -

produces a more preferred catalyst, comprises treating the solid catalyst with an aqueous basic wash solution after the first exposure to the nickel salt solution. Exposure to the nickel salt solution may then be repeated followed by another wash, and drying and calcination. The sequence of the exposure to $Ni^{+2}$ ions, washing and drying can be repeated several times before calcination. Base may optionally be used in any of the steps to increase the amount of reacted nickel. The preferred base is ammonium hydroxide. In another preferred embodiment, the nickel salt solution is heated to a temperature of from about 27°C to about 100°C. Such treatment serves to increase the number of sites available for the nickel cations to react within the $[SiO_2-Al_2O_3]$, and encourages the reaction of the nickel ion. The length of time for the exposure is not critical and may last for any period from the minimum of 30 seconds up to 48 hours.

As the concentration of the nickel salt solution increases the washing step must be done more carefully. When the nickel salt solution used is in excess of .3 molar, the washing step is preferably repeated so that the solid catalyst is well washed, removing substantially all of the unreacted nickel before it is dried and calcined.

After the solid catalyst is dried it is activated by calcination. Acceptably, this calcination step takes place by heating the solid catalyst to a temperature in the range of from about 300°C to about 700°C. A more preferable temperature range for calcination, however, is from about 400°C to about 600°C. The most preferred catalyst is calcined in a low pressure system where the pressure is in the range of from about

.l mm of Hg to about 340 mm of Hg, although an acceptable catalyst can be produced by pressures in the range of from about .1 mm of Hg to about 760 mm of Hg.

After the solid catalyst is prepared and activated, it can be used for olefin oligomerization. Typically, the process of olefin oligomerization takes place at such temperatures and pressures as will insure that a liquid-phase oligomerization is conducted. Olefins useful with this catalyst contain from 2 to about 12 carbon atoms. A preferred range of olefins useful with the catalysts of the present invention is from 2 to about 6 carbon atoms.

The solid catalyst can be used to oligomerize lightly branched and/or linear olefins by a process comprising: contacting the catalyst with lightly branched and/or linear olefins, at temperatures in the range of from about 20°C to about 400°C and under pressures in the range of from about 1 to about 75 atmospheres, and collecting the oligomerized product.

A suitable reaction temperature for this catalyst ranges from 20°C to 400°C, since for this catalyst, a more moderate temperature is capable of being used. A preferred temperature range is from about 20°C to about 200°C. The most preferred range is from 40°C to about 150°C.

The oligomerization products are separated and recovered by conventional methods such as fractional distillation, selective extraction, absorption, and processes of this nature. Any reaction diluent used and any unreacted olefin may be recycled.

More specifically, the olefin oligomerization is carried out by contacting the olefin in liquid-phase with the surface of the catalyst. While carrying out the oligomerization it may be convenient to use a diluent, although diluent use is not critical. If a diluent is used, the molar ratio of the diluent to the olefin can range up to 10 moles of diluent per mole of olefin. Representative but non-exhaustive examples of suitable diluents are alkanes, such as pentane, hexane, and heptane.

The invention is more concretely described with reference to the examples below wherein all parts and percentages are by weight unless otherwise specified. These examples are provided to illustrate the instant invention and not to limit it.

Example 1

20 grams (g) of 20-30 mesh silica-alumina (13 wt.% alumina) was combined with 100 grams of 15 weight percent (0.56 molar) aqueous solution of nickel (II) nitrate hexahydrate and 17 milliliters (ml) of 3.0 molar (M) ammonium hydroxide. The mixture was heated on a steam bath for 48 hours, following which the supernatant liquid was decanted. The particulate silica-alumina was washed thoroughly with several portions of water, then with 100 ml of 3.0 M ammonium hydroxide, and again with several portions of water. The solid silica-alumina was then combined with 150 g of a 10 weight percent (0.36 M) aqueous solution of nickel (II) nitrate hexahydrate, and the mixture was heated on a steam bath for 16 hours. The supernatant liquid was again decanted and the solid material was washed with several portions of water, after which the catalyst was again immersed in 150 g of 10 weight percent (o.36 M) nickel nitrate solution, after which the catalyst was washed thoroughly with several portions of water and dried for three hours at 110°C. The solid catalyst was calcined in a reduced pressure system of 0-5 mm of Hg for 6 hours at 510°C. The reduced pressure system contained a vacuum line cold trap which collected gases given off by the solid catalyst. Only a trace of nitrogen dioxide was collected in the cold trap during calcination which indicated that nickel ions had been incorporated on the silica-alumina support primarily by cationic change and were only to a slight extent present as nickel nitrate. After the catalyst cooled, 5.0 g of it was transferred under argon to a 100-ml autoclave which was then charged with 20 ml of n-hexane and 24.8 g of cis- and trans-2-butene (pure grade). The autoclave was pressurized to 100 psig with argon, and the reaction mixture was stirred magnetically at 26°C for 4.7 hours. The product was collected and hydrogenated. A subsequent analysis by gas chromatography (methyl silicone stationary phase, in a 50 meter (m) fused silica capillary column) showed that 41.5% of the butene was converted to a mixture of oligomers consisting of 75.3 weight percent dimer, 21.4 weight percent trimer and 3.3 weight percent tetramer. The hydrogenated dimer was made up of 61.7% 3,4-dimethylhexane; 29.8% 3-methylheptane; 5.7% n-oxtane; and 2.8% other structures. The average degree of branching in the dimer is calculated as 1.59.

Example 2

A catalyst was prepared in the same manner as described in Example 1 except that this catalyst was calcined in a flowing stream of argon for 16 hours at 500°C.

The catalyst was tested in the following manner: 25.2 g of cis- and trans-2-butene was put into a 100-ml autoclave along with 2.0 g of catalyst and 20 ml of n-hexane. The mixture was stirred magnetically at 50°C for 10 hours.

The product was hydrogenated and analysed as described in Example 1. The results of the analysis indicated that 25% of the butene was converted to an oligomer mixture consisting of: 89.3 weight percent dimer; 9.8 weight percent trimer; and 0.9 weight percent tetramer. Hydrogenated dimer consisted of 93.9% 3,4-dimethylhexane , 2.1% 3-methylheptane; 0.3% n-octane; and 3.8% other structures. The average degree of branching in the dimer was 1.98.

## Example 3
## Comparison Example Of An Impregnated Nickel Oxide On Silica-Alumina Catalyst

A nickel oxide on silica-catalyst was prepared as follows , 35 g of silica-alumina was soaked for 10 minutes in a 1.72 M solution of nickel (II) nitrate hexahydrate. The excess solution was removed by suction filtration, and the impregnated silica-alumina was dried overnight at 110°C, then calcined in air at 510°C for 16 hours. During the early stages of calcination, the evolution of substantial quantities of nitrogen dioxide was noted, as expected from the known decomposition of nickel nitrate to nickel oxide. Analysis of the calcined catalyst by atomic absorption spectroscopy showed it to contain 5.45 weight percent of nickel. A 5.0 g portion of this catalyst was transferred under argon to a 100 ml autoclave, which was then charged with 20 ml of n-hexane and 26.0 g of trans-2-butene. The contents of the autoclave were stirred magnetically and heated at 101°C for 6 hours. A 1.0 g sample of the product was hydrogenated. Subsequent analysis of the hydrogenated sample by gas chromatography (methyl silicone stationary phase, in a 50 m fused silica capillary column) showed a butene conversion of 44 percent, as calculated by use of the hexane solvent as internal standard. The same analysis showed the butene oligomer to contain 72.0 weight percent dimer, 25.6 weight percent trimer, and 2.5 weight percent tetramer. The hydrogenated dimer was composed of 68.8% 3,4-dimethylhexane; 14.8% 3-methylheptane; 4.7% n-octane; and 11.7% other structures. Assuming these other structures to be doubly-branched, the average degree of branching in the dimer is calculated to be 1.76.

As can be seen when comparing Example 1 with the comparison example 3 demonstrating an impregnation catalyst, the catalyst of the instant invention proved more active (Example 1 converted 41.5 wt% of butene in 4.7 hours at 26°C, whereas the impregnated catalyst in 6 hours at the much higher temperature of 101°C, converted 44 wt% of the butene). The catalysts of the instant invention are also distinguished from the impregnated catalyst of the comparison Example 3 in that the product has a higher weight percent dimer content.

While certain details have been shown for the purpose of illustrating this invention, it would be apparent to those skilled in this art that various changes and modifications may be made herein without departing from the spirit or scope of the invention.

Claims:

1. A process for the preparation of a non-impregnated, chemically incorporated $Ni^{+2}$ silica-alumina catalyst suitable for use in the oligomerization of olefins which comprises:

    (a) contacting silica-alumina $[SiO_2-Al_2O_3]$ with molar excess of $Ni^{+2}$ cations for a sufficient length of time to permit an essentially complete chemical reaction between $Ni^{+2}$ cations and the accessible reactive sites in the silica-alumina;

    (b) washing the solid catalyst of step (a) in an effective amount of water for a sufficient length of time to remove substantially all unbound $Ni^{+2}$ cations from step (a); and

    (c) drying and then calcining the solid catalyst remaining after step (b) by heating it to a temperature of from about 300°C to about 700°C.

2. A process as claimed in claim 1 wherein the solid silica-alumina is immersed in a nickel salt solution, the volume of nickel salt solution used being at least twice the volume of the silica-alumina used.

3. A process as claimed in claim 2 wherein the nickel salt solution used has a concentration of at least 0.05 molar.

4. A process as claimed in any one of the preceding claims wherein the solid catalyst of step (a) is washed in an effective amount of water for at least 30 seconds.

5. A process as claimed in any one of the preceding claims wherein the solid catalyst remaining after step (a) is washed in an aqueous ammonium hydroxide solution.

6. A process as claimed in claim 4 wherein the $Ni^{+2}$ ions are present in an aqueous solution with a sufficient amount of base to afford a pH in the range of from about 7.2 to about 12.

7.      A process as claimed in claim 6 wherein the aqueous basic solution is heated to a temperature in the range of from about 27°C to about 100°C for a period of time of from about 5 minutes to about 48 hours.

8.      A non-impregnated, chemically incorporated $Ni^{+2}$ silica-alumina catalyst suitable for use in the oligomerization of olefins whenever prepared by a process which includes the process steps claimed in any one of the preceding claims.

9.      Use of a catalyst as claimed in claim 8 in the oligomerization of olefins.